# EUROPEAN PATENT APPLICATION

(11) **EP 4 095 125 A1**
(43) Date of publication of application: **30.11.2022**
(21) Application number: 21744803.4
(22) Date of filing: 07.01.2021
(51) Int. Cl.: C07C 291/04, C07C 215/10

(54) **COMPOSITION AND METHOD FOR PRODUCING SAME**

(30) Priority: 22.01.2020 JP 2020008507
(71) Applicant: Daicel Corporation, Osaka 530-0011 (JP)
(72) Inventor: FUJII, Hiroyuki, Tokyo 108-8230 (JP)
(74) Representative: Grünecker Patent- und Rechtsanwälte PartG mbB
(86) International application number: PCT/JP2021/000386
(87) International publication number: WO 2021/149502

(57) **Abstract**

Provided is a composition having moderate viscosity for coating properties and ejection properties, being applicable for firing at low temperatures, and leaving an extremely small amount of ash after firing. The composition of the present disclosure contains a miscible material of a compound represented by Formula (1) below and a compound (A) represented by Formula (A) below. In Formula (1) below, R¹ represents a linear aliphatic hydrocarbon group having from 10 to 25 carbons; R² and R³ are identical or different and represent an aliphatic hydrocarbon group having 2, 4, 6, or 8 carbons; R⁴ represents an aliphatic hydrocarbon group having from 1 to 8 carbons; R⁵ and R⁶ are identical or different and represent an aliphatic hydrocarbon group having from 1 to 3 carbons or a hydroxyalkyl ether group; and L¹ to L³ represent an amide bond. In Formula (A) below, in the formula, R^{a} and R^{c} are identical or different and represent a hydrogen atom or an aliphatic hydrocarbon group that has from 1 to 12 carbons and may have a substituent; R^{b} represents an aliphatic hydrocarbon group that has from 1 to 12 carbons and may have a substituent; and the substituents are each an amino group and/or a hydroxyl group.

## Description

### Technical Field

The present disclosure relates to a composition used during production of an electronic device and a method for producing the composition. The present application claims priority to JP 2020-008507 filed in Japan on January 22, 2020, the content of which is incorporated herein.

### Background Art

Electronic devices produced using a printing method include printed wiring boards, capacitors, inductors, varistors, thermistors, transistors, speakers, actuators, antennas, solid oxide fuel cells, and hybrid integrated circuits (ICs).

For example, a multilayer ceramic capacitor is typically produced through a process as follows:
1. forming a slurry containing a ceramic powder; a binder resin, such as a poly(vinyl acetal) resin; and a solvent, into a sheet form, and producing a green sheet;
2. applying a conductive metal paste containing an electrical property-imparting material (e.g., such as nickel or palladium), a binder resin (e.g., such as ethyl cellulose), and a fluid organic material (e.g., such as terpineol) onto the green sheet by a printing method and forming a wiring or an electrode of a conductive circuit (which may be hereinafter referred to as a "wiring or the like") (application);
3. drying the applied conductive metal paste (drying);
4. cutting the green sheet on which the wiring or the like is formed to a predetermined size and laminating and crimping a plurality of sheets; and
5. firing the laminate (firing).

The binder resin contained in the conductive metal paste has a function to fix the electrical property-imparting material on the green sheet and a function to impart moderate viscosity to the paste to enable formation of a fine printed pattern. For the binder resin, ethyl cellulose has been mainly used in the related art. However, ethyl cellulose has poor thermal decomposition properties and thus firing needs to be performed at high temperature. Exposure to high temperature for a long time may cause softening or deformation of an object coated with the paste, and cause a carbon component to remain as ash after firing, and this may reduce the conductivity, which have been problems with ethyl cellulose.

To solve the above problems, various improvements in the binder resin have been investigated. For example, Patent Document 1 discloses that use of a poly(vinyl acetal) resin instead of ethyl cellulose can reduce the amount of ash produced. However, the use of a poly(vinyl acetal) resin has not yet achieved a sufficiently satisfactory result for these problems.

### Citation List

### Patent Document

Patent Document 1: JP 2006-299030 A

### Summary of Invention

### Technical Problem

Thus, an object of the present disclosure is to provide a composition for forming a wiring and an electrode of an electronic device by a printing method, the composition having moderate viscosity for coating properties and ejection properties suitable for the printing method, being applicable for firing at low temperatures, and leaving an extremely small amount of ash after firing; and to provide a method for producing the composition.

### Solution to Problem

As a result of diligent research to solve the above problems, the inventor of the present disclosure found that a composition containing a miscible material of a compound (1) represented by Formula (1) below and a compound (A), a specific fluid organic material, represented by Formula (A) below exhibits moderate viscosity and stabilizes (preventing the precipitation, local aggregation, or condensation of the composition, and stably maintaining a uniform state) even when the content of the compound (1) is small; and the composition can be fired at lower temperatures compared to a binder resin, such as ethyl cellulose, and residual amount of ash after firing can be extremely reduced. The invention according to the present disclosure was completed based on these findings.

That is, the present disclosure provides a composition containing a miscible material of:
a compound (1) and a compound (A), the compound (1) being represented by Formula (1) below: 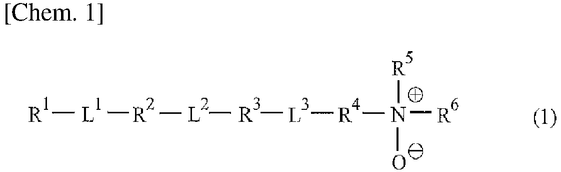
where R¹ represents a monovalent linear aliphatic hydrocarbon group having from 10 to 25 carbons; R² and R³ are identical or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbons; R⁵ and R⁶ are identical or different and represent a monovalent aliphatic hydrocarbon group having from 1 to 3 carbons or a hydroxyalkyl ether group; L¹ to L³ represent an amide bond; when L¹ and L³ are -CONH-, L² is -NHCO-; and when L¹ and L³ are -NHCO-, L² is -CONH-; and
the compound (A) being represented by Formula (A) below: 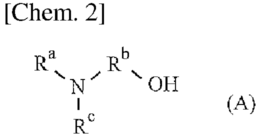
where R^{a} and R^{c} are identical or different and represent a hydrogen atom or a monovalent aliphatic hydrocarbon group that has from 1 to 12 carbons and may have a substituent; R^{b} represents a divalent aliphatic hydrocarbon group that has from 1 to 12 carbons and may have a substituent; and the substituents are each an amino group and/or a hydroxyl group.

The present disclosure also provides the composition, in which R^{c} in Formula (A) above is a hydrogen atom

The present disclosure also provides the composition, in which the compound (A) is a compound (A') represented by Formula (A') below: 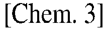 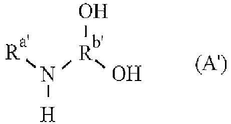 where R^{a'} represents a monovalent aliphatic hydrocarbon group having from 1 to 12 carbons; and R^{b'} represents a trivalent aliphatic hydrocarbon group having from 1 to 12 carbons.

The present disclosure also provides the composition further containing an electrical property-imparting material.

The present disclosure also provides a method for producing a composition, in which the composition described above is formed through mixing a compound (1) represented by Formula (1) below and a compound (A) represented by Formula (A) below to form a miscible material: 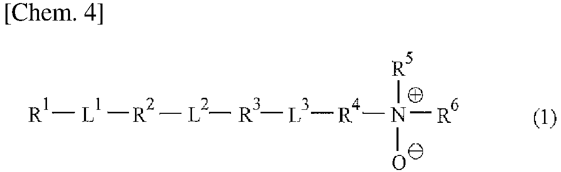 where R¹ represents a monovalent linear aliphatic hydrocarbon group having from 10 to 25 carbons; R² and R³ are identical or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbons; R⁵ and R⁶ are identical or different and represent a monovalent aliphatic hydrocarbon group having from 1 to 3 carbons or a hydroxyalkyl ether group; L¹ to L³ represent an amide bond; when L¹ and L³ are -CONH-, L² is -NHCO-; and when L¹ and L³ are -NHCO-, L² is -CONH-; and 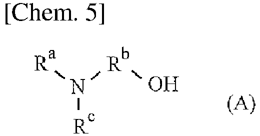 where R^{a} and R^{c} are identical or different and represent a hydrogen atom or a monovalent aliphatic hydrocarbon group that has from 1 to 12 carbons and may have a substituent; R^{b} represents a divalent aliphatic hydrocarbon group that has from 1 to 12 carbons and may have a substituent; and the substituents are each an amino group and/or a hydroxyl group.

### Advantageous Effects of Invention

The composition of the present disclosure contains the miscible material of the specific compound (1) and the compound (A), a specific fluid organic material, and the combination of the compound (1) and the compound (A) exhibits moderate viscosity even when the content of the compound (1) is small. Thus, the composition is less susceptible to dripping and has good coating properties (or ejection properties). In addition, the composition can form a drawing part with a clearly defined edge and thus can improve the printing precision. Furthermore, the composition of the present disclosure can stably maintain the uniformity of the composition. For example, when the composition of the present disclosure contains an electrical property-imparting material, the composition can prevent the precipitation and local aggregation of the electrical property-imparting material and can stably maintain a uniformly and highly dispersed state of the electrical property-imparting material. Moreover, the content of the compound (1) can be reduced, and thus, compared to a composition in the related art obtained by thickening a fluid organic material with ethyl cellulose, the composition of the present disclosure can be fired at a lower temperature. This can prevent an object coated with the composition from softening and deformation due to exposure to high temperature for a long time. Moreover, a residual amount of ash after firing can be significantly reduced, and this can prevent various problems that have been caused by residual ash (e.g., such as reduction in electrical properties).

### Brief Description of Drawings

FIG. 1 is a diagram showing a ¹H-NMR measurement result of a compound (2-3) obtained in a preparation example.
FIG. 2 is a diagram showing a ¹H-NMR measurement result of a compound (1-3) obtained in a preparation example.
FIG. 3 is a diagram showing a ¹H-NMR measurement result of a compound (2-4) obtained in a preparation example.
FIG. 4 is a diagram showing a ¹H-NMR measurement result of a compound (1-4) obtained in a preparation example.
FIG. 5 is a diagram showing a ¹H-NMR measurement result of a compound (2-1) obtained in a preparation example.
FIG. 6 is a diagram showing a ¹H-NMR measurement result of a compound (1-1) obtained in a preparation example.

### Description of Embodiments

### Composition

A composition of the present disclosure is a composition that can be suitably used for producing electronic devices and a composition that can be suitably used in conductive metal pastes, conductive inks, conductive adhesives, and the like. The electronic devices include printed wiring boards, capacitors, inductors, varistors, thermistors, transistors, speakers, actuators, antennas, solid oxide fuel cells, and hybrid ICs. The composition of the present disclosure is a composition containing a miscible material of a compound (1) and a compound (A), a fluid organic material.

### Compound (1)

The compound (1) is represented by Formula (1) below: 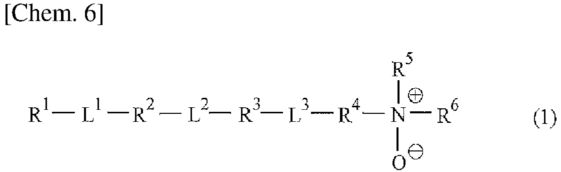 where R¹ represents a monovalent linear aliphatic hydrocarbon group having from 10 to 25 carbons; R² and R³ are identical or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbons; R⁵ and R⁶ are identical or different and represent a monovalent aliphatic hydrocarbon group having from 1 to 3 carbons or a hydroxyalkyl ether group; L¹ to L³ represent an amide bond; when L¹ and L³ are -CONH-, L² is -NHCO-; and when L¹ and L³ are -NHCO-, L² is -CONH-.

R¹ is a monovalent linear aliphatic hydrocarbon group having from 10 to 25 carbons, and examples include linear alkyl groups, such as a decyl group, a lauryl group, a myristyl group, a pentadecyl group, a stearyl group, a palmityl group, a nonadecyl group, an eicosyl group, and behenyl group; linear alkenyl groups, such as a decenyl group, a pentadecenyl group, an oleyl group, and an eicosenyl group; and linear alkynyl groups, such as a pentadecynyl group, an octadecynyl group, and a nonadecynyl group.

R¹ is a monovalent linear aliphatic hydrocarbon group having from 10 to 25 carbons, and from the viewpoints of excellent thickening effect of the fluid organic material and capability of reducing the residual ash to an extremely small amount even when the composition is fired at low temperature, R¹ is preferably a monovalent linear aliphatic hydrocarbon group having from 11 to 21 carbons (especially preferably a linear alkyl group having from 11 to 21 carbons) and more preferably a monovalent linear aliphatic hydrocarbon group having from 11 to 13 or from 17 to 21 carbons (especially preferably a linear alkyl group having from 11 to 13 or from 17 to 21 carbons).

Examples of the divalent aliphatic hydrocarbon group having 2, 4, 6, or 8 carbons for R² and R³ include an ethylene group, a n-butylene group, a n-hexylene group, and a n-octylene group.

Examples of the divalent alicyclic hydrocarbon group having 6 carbons for R² and R³ include a 1,4-cyclohexylene group, a 1,3-cyclohexylene group, and a 1,2-cyclohexylene group.

Examples of the divalent aromatic hydrocarbon group for R² and R³ include an arylene group having from 6 to 10 carbons, such as a 1,4-phenylene group, a 1,3-phenylene group, and a 1,2-phenylene group.

In particular, R² and R³ are, from the viewpoint of excellent viscosity-imparting effect, preferably a divalent aliphatic hydrocarbon group (especially preferably a linear alkylene group) having 2, 4, 6, or 8 carbons, more preferably a divalent aliphatic hydrocarbon group (especially preferably a linear alkylene group) having 2, 4, or 6 carbons, particularly preferably a divalent aliphatic hydrocarbon group (especially preferably a linear alkylene group) having 2 or 4 carbons, and most preferably a divalent aliphatic hydrocarbon group (especially preferably a linear alkylene group) having 2 carbons.

R⁴ represents a divalent aliphatic hydrocarbon group having from 1 to 8 carbons, and among these, from the viewpoint of excellent viscosity-imparting effect, R⁴ is preferably a linear or branched alkylene group and particularly preferably a linear alkylene group.

A combination of the number of carbons of the monovalent linear aliphatic hydrocarbon group for R¹ and the number of carbons of the divalent linear aliphatic hydrocarbon group for R⁴ is preferably a combination of R¹ having from 10 to 25 carbons and R⁴ having from 1 to 8 carbons and more preferably a combination of R¹ having from 11 to 21 carbons and R⁴ having from 1 to 8 carbons.

In addition, the combination of the numbers of carbons for R¹ and R⁴ is even more preferably a combination of R¹ having from 11 to 13 carbons and R⁴ having from 4 to 8 carbons or a combination of R¹ having from 17 to 21 carbons and R⁴ having from 1 to 8 carbons.

Furthermore, the combination of the numbers of carbons for R¹ and R⁴ is particularly preferably a combination of R¹ having from 11 to 13 carbons and R⁴ having from 4 to 8 carbons, a combination of R¹ having 17 carbons and R⁴ having from 1 to 5 carbons, or a combination of R¹ having from 18 to 21 carbons and R⁴ having from 4 to 8 carbons.

Moreover, the combination of the numbers of carbons for R¹ and R⁴ is most preferably a combination of R¹ having from 18 to 21 carbons and R⁴ having from 4 to 8 carbons.

Examples of the monovalent aliphatic hydrocarbon group having from 1 to 3 carbons for R⁵ and R⁶ include: linear or branched alkyl groups having from 1 to 3 carbons, such as a methyl group, an ethyl group, a propyl group, and an isopropyl group; linear or branched alkenyl groups having 2 or 3 carbons, such as a vinyl group, a 1-methylvinyl group, and 2-propenyl group; and linear or branched alkynyl groups having 2 or 3 carbons, such as an ethynyl group and a propynyl group.

Examples of the hydroxyalkyl ether group for R⁵ and R⁶ include a mono- or di- (hydroxy)C₁₋₃ alkyl ether group, such as a 2-hydroxyethoxy group, a 2-hydroxypropoxy group, and a 2,3-dihydroxypropoxy group.

In particular, R⁵ and R⁶ are identical or different, and preferably a monovalent aliphatic hydrocarbon group having from 1 to 3 carbons, more preferably a linear or branched alkyl group having from 1 to 3 carbons, particularly preferably a linear alkyl group having from 1 to 3 carbons, and especially preferably a methyl group.

The compound (1) is among others preferably a compound represented by Formulas (1-1) to (1-9) below from the viewpoints of excellent miscibility with the fluid organic material (compound (A)) and in that a transparent composition can be obtained when the fluid organic material is transparent.

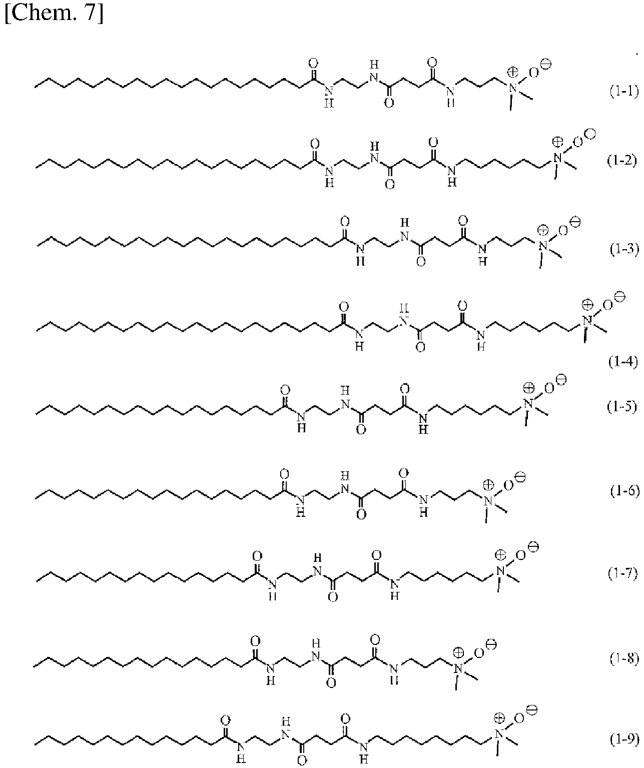

### Method for producing compound (1)

The compound (1) can be produced as follows: forming a compound represented by Formula (2) below (which may be hereinafter referred to as a "compound (2)") by reacting a compound represented by Formula (3) below (which may be hereinafter referred to as a "compound (3)") and a compound represented by Formula (4) below (which may be hereinafter referred to as a "compound (4)") or by reacting a compound represented by Formula (3') below (which may be hereinafter referred to as a "compound (3')") and a compound represented by Formula (4') below (which may be hereinafter referred to as a "compound (4')"); and oxidizing the resulting compound (2). 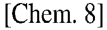 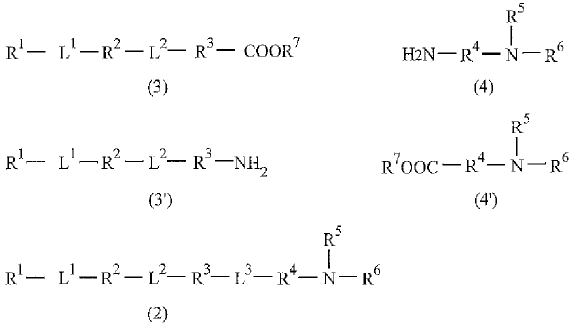 where R¹ represents a monovalent linear aliphatic hydrocarbon group having from 10 to 25 carbons; R² and R³ are identical or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbons; R⁵ and R⁶ are identical or different and represent a monovalent aliphatic hydrocarbon group having from 1 to 3 carbons or a hydroxyalkyl ether group; L¹ to L³ represent an amide bond; when L¹ and L³ are -CONH-, L² is -NHCO-; and when L¹ and L³ are -NHCO-, L² is -CONH-; R⁷ represents a hydrogen atom or a alkyl group having from 1 to 3 carbons; and in Formula (3), OR⁷ may form a ring through dehydration condensation or dealcoholization condensation with a hydrogen atom constituting L².

R¹ to R⁶ and L¹ and L² in the above formulas are the same as described above.

Examples of the alkyl group having from 1 to 3 carbons for R⁷ in Formulas (3) and (4') above include a methyl group, an ethyl group, a propyl group, and an isopropyl group.

Examples of the ring formed through dehydration condensation or dealcoholization condensation of OR⁷ in Formula (3) with a hydrogen atom constituting L² include a pyrrolidine-2,5-dione ring and a piperidine-2,6-dione ring.

The compound (4) is used in any amount of 1 mol or higher per mol of the compound (3) and can also be used in an excess amount.

The compound (4') is used in any amount of 1 mol or higher per mol of the compound (3') and can also be used in an excess amount.

The reaction of the compound (3) and the compound (4) or the reaction of the compound (3') and the compound (4') can be carried out, for example, by stirring at a temperature of 100 to 120°C for 10 to 20 hours.

A reaction atmosphere is any atmosphere that does not inhibit the reaction and not particularly limited, and may be, for example, any of an air atmosphere, a nitrogen atmosphere, and an argon atmosphere. In addition, the reaction can be carried out by any method, such as a batch method, a semi-batch method, and a continuous method.

After completion of the reaction, the resulting reaction product can be separated and purified by a separation means, such as filtration, concentration, distillation, extraction, crystallization, adsorption, recrystallization, or column chromatography; or a separation means in combination of these.

The compound (2) thus obtained is among others preferably a compound represented by Formulas (2-1) to (2-9) below.

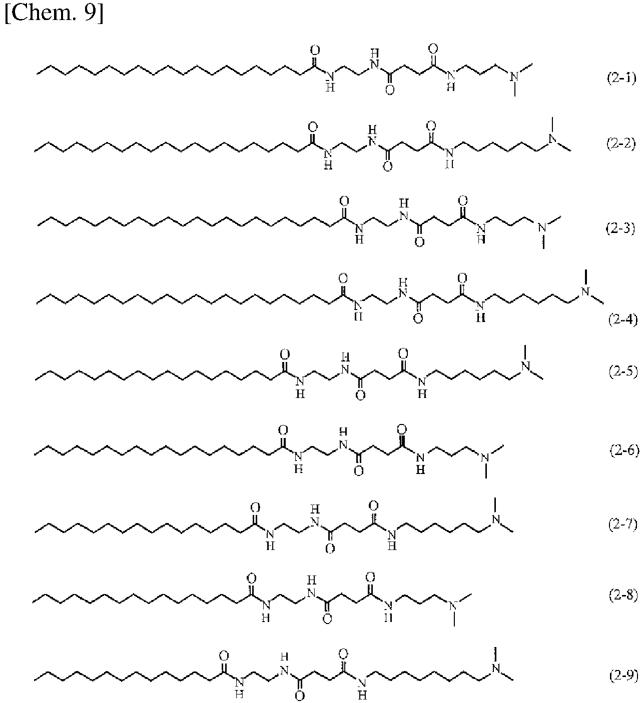

The compound (3), for example, a compound represented by Formula (3-1) below can be produced by a method described below. R¹, R², R³, and R⁷ in the formula below are the same as described above. In addition, R⁷ in Formula (3a) and two R⁷s in Formula (3d) may be identical to or different from each other. Furthermore, the compound represented by Formula (3d) may be an anhydride formed by dehydration condensation of two COOR⁷s in the formula.

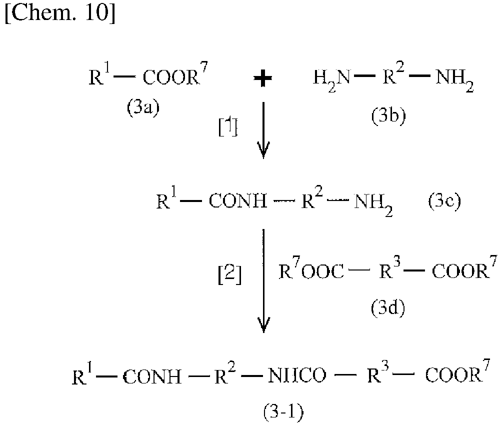

In addition, the compound (3'), for example, a compound represented by Formula (3'-1) below can be produced by a method described below. R¹, R², R³, and R⁷ in the formula below are the same as described above. Two R⁷s in Formula (3b') may be identical or different. Furthermore, the compound represented by Formula (3b') may be an anhydride formed by dehydration condensation of two COOR⁷s in the formula.

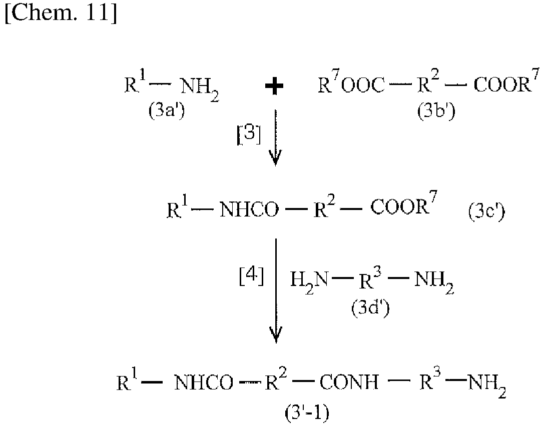

A process [1] is to react a compound represented by Formula (3a) and a compound represented by Formula (3b) to yield a compound represented by Formula (3c). The compound represented by Formula (3b) is used in any amount of 1 mol or higher per mol of the compound represented by Formula (3a) and can also be used in an excess amount. A reaction temperature of this reaction is, for example, from 80 to 150°C, and the reaction time is, for example, approximately from 1 to 24 hours.

A process [2] is to react the compound represented by Formula (3c) and a compound represented by Formula (3d) to yield the compound represented by Formula (3-1). The compound represented by Formula (3d) is used in any amount of 1 mol or higher per mol of the compound represented by Formula (3c) and preferably of 1 to 3 mol. A reaction temperature of this reaction is, for example, from 80 to 150°C, and the reaction time is, for example, approximately from 0.5 to 10 hours. As this reaction proceeds, water is produced. Thus, the reaction is preferably carried out while water is being removed using a dehydrating agent (e.g., such as acetic anhydride) to promote the progress of the reaction.

The reaction in [2] is preferably carried out in the presence of a solvent. Examples of the solvent include pentafluorophenol, N,N-dimethylformamide, dimethylacetamide, and o-dichlorobenzene. One of these can be used alone or two or more in combination.

In addition, the reaction in [2] can be carried out in the presence of a base, such as triethylamine, pyridine, or 4-dimethylaminopyridine, as necessary.

A process [3] is to react a compound represented by Formula (3a') and the compound represented by Formula (3b') to yield a compound represented by Formula (3c'). The reaction in [3] can be carried out under the conditions similar to those for the reaction in [2] above.

A process [4] is to react the compound represented by Formula (3c') and a compound represented by Formula (3d') to yield the compound represented by Formula (3'-1). The reaction in [4] can be carried out under the conditions similar to those for the reaction in [1] above.

After completion of the reaction in each process, the resulting reaction product can be separated and purified by a separation means, such as filtration, concentration, distillation, extraction, crystallization, adsorption, recrystallization, or column chromatography; or a separation means in combination of these.

For the oxidizing agent used for the oxidation of the compound (2) formed by the method described above, for example, hydrogen peroxide can be used. For the hydrogen peroxide above, pure hydrogen peroxide may be used, but from the viewpoint of handling, the hydrogen peroxide is typically diluted in an appropriate solvent (e.g., water) and used (e.g., an aqueous solution from 5 to 70 wt.% hydrogen peroxide). Hydrogen peroxide is used in an amount, for example, approximately of 0.1 to 10 mol per mol of the compound (2).

The oxidation reaction can be carried out, for example, by stirring at a temperature of 30 to 70°C for 3 to 20 hours.

The oxidation reaction of the compound (2) is carried out in the presence or absence of a solvent. Examples of the solvent include an alcohol-based solvents, such as methanol, ethanol, 2-propanol, and butanol; ether-based solvents, such as diethyl ether, diisopropyl ether, dibutyl ether, tetrahydrofuran, dioxane, dioxolane, 1,2-dimethoxyethane, and cyclopentyl methyl ether; ester-based solvents, such as butyl acetate and ethyl acetate; hydrocarbon-based solvents, such as pentane, hexane, heptane, and octane; and nitrile-based solvents, such as acetonitrile and benzonitrile. One of these can be used alone or two or more in combination.

After completion of the reaction, the resulting reaction product can be separated and purified by a separation means, such as filtration, concentration, distillation, extraction, crystallization, adsorption, recrystallization, or column chromatography; or a separation means in combination of these.

### Fluid organic material

In the composition of the present disclosure, the compound (A) represented by Formula (A) below is used as the fluid organic material. 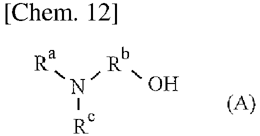 where R^{a} and R^{c} are identical or different and represent a hydrogen atom or a monovalent aliphatic hydrocarbon group that has from 1 to 12 carbons and may have a substituent; R^{b} represents a divalent aliphatic hydrocarbon group that has from 1 to 12 carbons and may have a substituent; and the substituents are each an amino group and/or a hydroxyl group.

Examples of the monovalent aliphatic hydrocarbon group having from 1 to 12 carbons for R^{a} and R^{c} in Formula (A) above include linear or branched alkyl groups, such as, for example, a methyl group, an ethyl group, a propyl group, an isopropyl group, a butyl group, an isobutyl group, a sec-butyl group, a tert-butyl group, a pentyl group, an isopentyl group, a sec-pentyl group, a tert-pentyl group, a neopentyl group, a 3- pentyl group, a hexyl group, a heptyl group, an octyl group, a 2-ethylhexyl group, a nonyl group, a decyl group, an undecyl group, and a dodecyl group.

Among these, R^{a} and R^{c} are preferably a monovalent aliphatic hydrocarbon group having from 1 to 8 (preferably from 1 to 4) carbons, more preferably an alkyl group having from 1 to 8 (preferably from 1 to 4) carbons, and even more preferably a methyl group, an ethyl group, or a propyl group.

In addition, R^{c} is preferably a hydrogen atom.

Examples of the divalent aliphatic hydrocarbon group having from 1 to 12 carbons for R^{b} in Formula (A) above include linear or branched alkylene groups, such as, for example, a methylene group, an ethylene group, an ethylidene group, a trimethylene group, a propylene group, a propylidene group, an isopropylidene group, a tetramethylene group, a butylidene group, an isobutylidene group, a sec-butylidene group, a pentylene group, an isopentylene group, a hexylene group, an isohexylene group, a heptylene group, an isoheptylene group, an octylene group, a 2-ethylhexylene group, a nonylene group, an isononylene group, a decylene group, an isodecylene group, an undecylene group, an isounedecylene group, a dodecylene group, and an isododecylene group.

Among these, R^{b} is preferably a divalent aliphatic hydrocarbon group having from 1 to 8 (preferably from 1 to 4) carbons, more preferably an alkylene group having from 1 to 8 (preferably from 1 to 4) carbons, even more preferably an ethylene group, a trimethylene group, or a tetramethylene group, and particularly preferably a trimethylene group.

The total number of amino groups and/or hydroxyl groups that may be contained in R^{a}, R^{b}, or R^{c} is preferably from 0 to 6, more preferably from 0 to 4, and even more preferably from 0 to 2.

Examples of the compound (A) include amino-alkanols, such as 2-aminoethanol, 3-aminopropanol, 1-amino-2-propanol, 1-amino-2-methyl-2-propanol, 2-amino-2-methyl-1-propanol, 4-amino-1-butanol, 6-amino-1-hexanol, 10-amino-1-decanol, and 12-amino-1-dodecanol; monoalkylamino-alkanols, such as N-methyl-2-aminoethanol, N-ethyl-2-aminoethanol, and N-propyl-2-aminoethanol; dialkylamino-alkanols, such as 2-dimethylaminoethanol and 6-diethylaminohexanol; trialkanol)amines, such as triethanolamine, tri-n-propanolamine, triisopropanolamine, tri-n-butanolamine, and triisobutanolamine; di(alkanol)amines, such as diethanolamine, di-n-propanolamine, diisopropanolamine, di-n-butanolamine, and diisobutanolamine; amino-alkanediols, such as 1-amino-2,3-propanediol, 4-amino-1,2-butanediol, 4-amino-1,3-butanediol, 2-amino-1,3-propanediol, and 2-amino-2-methyl-1,3-propanediol; monoalkylamino-alkanediols, such as 1-methylamino-2,3-propanediol, 1-ethylamino-2,3-propanediol, 1-propylamino-2,3-propanediol, and 1-butylamino-2,3-propanediol; dialkylamino-alkanediols, such as 3-dimethylamino-1,2-propanediol and 2-diethylamino-1,3-propanediol; diaminoalkanols, such as 1,3-diaminopropan-2-ol; (aminoalkyl)amino-alkanols, such as 2-(2-aminoethylamino)ethanol and 2-(2-aminopropylamino)ethanol; and (aminoalkyl)(alkyl)amino-alkanols, such as 2-(2-aminoethylmethylamino)ethanol. One of these can be used alone or two or more in combination.

Among these, in terms of facilitating providing thickening effect, an amino-alkanediol or a monoalkylamino-alkanediol, which is a compound (A') represented by Formula (A') below, is preferred. 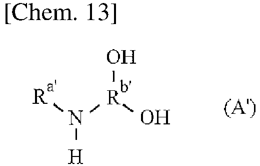 where R^{a'} represents a monovalent aliphatic hydrocarbon group having from 1 to 12 carbons; and R^{b'} represents a trivalent aliphatic hydrocarbon group having from 1 to 12 carbons.

Examples of the monovalent aliphatic hydrocarbon group having from 1 to 12 carbons according to R^{a'} above include the same as those for R^{a} in Formula (A) above.

Examples of the trivalent aliphatic hydrocarbon group having from 1 to 12 carbons according to R^{b'} above include a methylidyne group, an ethylidyne group, a propylidyne group, a butylidyne group, and a pentylidyne group.

Among the compounds (A'), 1-amino-2,3-propanediol or 1-methylamino-2,3-propanediol is more preferred.

### Method for producing composition

The composition of the present disclosure can be produced through mixing the compound (1) and the compound (A) to form a miscible material. More in detail, the composition can be produced by mixing and warming the total amount of the compound (A) and the compound (1) to form a miscible material, and then cooling. In addition, the composition can also be produced by a method of mixing the compound (1) in a portion of the compound (A), warming the mixture to form a miscible material, then cooling the miscible material, and mixing this in the rest of the compound (A).

The temperature in mixing to form a miscible material is appropriately selected according to the types of compound (1) and compound (A) and is any temperature at which the compound (1) and the compound (A) are mixed to form a miscible material, and is not particularly limited. But the temperature preferably does not exceed 100°C. When the boiling point of the compound (A) is 100°C or lower, the temperature is preferably at or around the boiling point.

The cooling after mixing to form a miscible material is any measure that can cool the miscible material to room temperature (e.g., 25°C); the miscible material may be gradually cooled at room temperature or rapidly cooled by ice cooling or the like.

Although the amount of the compound (1) depends on the type of compound (A), the compound (1) is used in an amount, for example, from 0.1 to 50 parts by weight, preferably from 0.5 to 20 parts by weight, particularly preferably from 1 to 10 parts by weight, and most preferably from 1 to 7 parts by weight per 1000 parts by weight of the compound (A). Using the compound (1) in the above range can provides a uniformly stabilized composition having moderate viscosity.

The content of the miscible material (or the total amount of the compound (1) and the compound (A)) in the total amount (100 wt.%) of the composition of the present disclosure is not particularly limited, but in a case where the composition serves as a composition for a conductive metal paste, the content is, for example, approximately from 0.1 to 30 wt.%, preferably from 0.1 to 20 wt.%, more preferably from 0.1 to 10 wt.%, even more preferably from 0.1 to 5 wt.%, and particularly preferably from 0.1 to 3 wt.%, and in a case where the composition serves as a composition other than a composition for a conductive metal paste, the content is, for example, approximately from 30 to 99.9 wt.%, preferably from 50 to 99.9 wt.%, more preferably from 60 to 99.9 wt.%, even more preferably from 70 to 99.9 wt.%, particularly preferably from 90 to 99.9 wt.%, and most preferably from 97 to 99.9 wt.%.

The composition of the present disclosure preferably contains an electrical property-imparting material (e.g., at least one selected from a conductive metal material, a semiconductor material, a magnetic material, a dielectric material, and an insulating material) as an additional component.

For the conductive metal material or the magnetic material, a material known and commonly used can be used, and examples can include gold, silver, copper, nickel, palladium, aluminum, iron, platinum, molybdenum, tungsten, zinc, lead, cobalt, iron oxide-chromium oxide, ferrite, and their alloys. For the semiconductor material, a material known and commonly used can be used, and examples can include pentacene, fullerene derivatives, polythiophene derivatives, metals (copper, indium, gallium, selenium, arsenic, cadmium, tellurium, and their alloys), and silicon fine particles. For the dielectric material or the insulating material, a material known and commonly used can be used, and examples can include cycloolefin polymers, fluorocarbon resins, butyral resins, glass, paper, and Teflons (trade name).

In a case where the composition serves as a composition for a conductive metal paste, the content of the conductive metal material or the magnetic material (the total amount when two or more are contained) is, for example, from 70 to 99.9 wt.%, preferably from 80 to 99.9 wt.%, more preferably from 90 to 99.9 wt.%, even more preferably from 95 to 99.9 wt.%, and particularly preferably from 97 to 99.9 wt.% of the total amount (100 wt.%) of the composition.

In a case where the composition serves as a composition other than a composition for a conductive metal paste, such as, for example, a composition for a conductive ink or a conductive adhesive, the content of the electrical property-imparting material (the total amount when two or more are contained) is, for example, from 0.1 to 30 wt.%, preferably from 0.1 to 20 wt.%, more preferably from 0.1 to 10 wt.%, even more preferably from 0.1 to 5 wt.%, and particularly preferably from 0.1 to 3 wt.% of the total amount (100 wt.%) of the composition.

In addition, viscosity [viscosity (η) at 25°C and a shear rate of 0.3 s⁻¹] of the composition of the present disclosure measured with a rheometer is preferably in a range of 10 Pa·s or higher (e.g., from 10 to 100 Pa·s) in that this can prevent dripping or flowing of the coating composition and improve the coating precision. Furthermore, the viscosity in the above range is preferred in that this can stably maintain the uniformity of the composition and in that in a case where the composition contains an electrical property-imparting material, the composition can prevent the precipitation and local aggregation of the electrical property-imparting material and can stably maintain a uniformly and highly dispersed state of the electrical property-imparting material.

Moreover, the viscosity [viscosity (η) at 25°C and a shear rate of 0.1 s⁻¹] of the composition of the present disclosure measured with a rheometer is preferably in a range of 10 Pa·s or higher (e.g., from 10 to 100 Pa·s) in that this can prevent dripping or flowing of the coating composition and improve the coating precision. Still more, the viscosity in the above range is preferred in that this can stably maintain the uniformity of the composition and in that in a case where the composition contains an electrical property-imparting material, the composition can prevent the precipitation and local aggregation of the electrical property-imparting material and can stably maintain a uniformly and highly dispersed state of the electrical property-imparting material.

The composition of the present disclosure has shear-thinning properties, and the viscosity ratio [ratio of the viscosity at 25°C and a shear rate of 1 s⁻¹/the viscosity at 25°C and a shear rate of 10 s⁻¹] measured with a rheometer is, for example, higher than 1.5, preferably 2 or higher, and particularly preferably 3 or higher. The upper limit is, for example, 10 and preferably 8. Thus, this can reduce the viscosity during coating and, for example, provides excellent ejection properties when the composition is coated using a printing machine or the like. Moreover, this rapidly increases the viscosity after coating and thus can prevent dripping of the coated composition and improve the coating precision.

The composition of the present disclosure has moderate viscosity, and thus this eliminates the need for adding a binder resin (e.g., a macromolecular compound with a molecular weight of 10000 or greater, such as an ethyl cellulose resin, an alkyl cellulose resin, a poly(vinyl acetal) resin, or an acrylic resin). Even in a case where the binder resin is added, the amount of the binder resin can be reduced, for example, to 10 wt.% or lower and preferably to 5 wt.% or lower of the total amount (100 wt.%) of the composition. When the amount of the binder resin added exceeds the above range, ash produced deriving from the binder resin by firing would reduce the electrical properties; thus, this is not preferred.

Furthermore, the miscible material contained in the composition of the present disclosure has excellent thermal decomposition properties and thus is easily decomposed into low molecular weight components. Thus, the composition of the present disclosure can be fired at lower temperatures (e.g., from 100 to 350°C, preferably from 150 to 300°C, and particularly preferably from 150 to 250°C) compared to a composition having a viscosity imparted by a binder resin, such as ethyl cellulose, and this can prevent the object coated with the composition from softening and deformation during firing.

The composition of the present disclosure combines the above properties and thus can be ejected well to a surface of a substrate (e.g., such as a ceramic substrate or a green sheet), for example, by screen printing, and this further forms a drawing part with a clearly defined edge and improve the printing precision. In addition, in a case where the composition of the present disclosure contains an electrical property-imparting material, the composition prevents the precipitation and local aggregation of the electrical property-imparting material and stably maintains a uniformly and highly dispersed state of the electrical property-imparting material and can be ejected and printed in that state, and drying and firing the ejected composition can form a wiring or the like with excellent electrical properties (e.g., conductivity or insulating properties) with good precision.

Thus, the composition of the present disclosure is particularly useful as a composition for conductive metal pastes and a composition for conductive inks used, for example, in printed wiring boards, such as multilayer printed wiring boards; capacitors, such as multilayer ceramic capacitors; inductors; varistors; thermistors; transistors; speakers; actuators; antennas; solid oxide fuel cells (SOFCs); and hybrid ICs; and particularly in multilayer ceramic capacitors.

In addition, the composition of the present disclosure can be ejected selectively, for example, by screen printing, to a desired position on the surface of the substrate provided with an electrode, a circuit, or the like, then an electronic component or the like can be bonded to the substrate and the composition can be fired, and thus the substrate and the electronic component can be electrically connected. Furthermore, the compound of the present disclosure can be fired at a low temperature, thus component implementation can be performed at a lower temperature than component implementation employing soldering. Thus, the compound of the present disclosure can be used for implementation of an electronic component and the like having poor heat resistance.

Thus, the composition of the present disclosure is particularly useful as a conductive adhesive used in producing, for example, printed wiring boards, such as multilayer printed wiring boards; capacitors, such as multilayer ceramic capacitors; inductors; varistors; thermistors; transistors; speakers; actuators; antennas; solid oxide fuel cells (SOFCs); and hybrid ICs; and particularly multilayer ceramic capacitors.

Each of the configurations, their combinations, and the like of the present disclosure above is an example, and an addition, an omission, a substitution, and a change of the configuration can be appropriately made without departing from the gist of the present disclosure. In addition, the present disclosure is not limited by the embodiments and is limited only by the claims.

### Examples

Hereinafter, the invention according to the present disclosure will be described more specifically through examples, but the invention according to the present disclosure is not limited by these examples.

### Preparation Example 1: Production of compound (1-3)

Methyl docosanoate (20.0 g, 56.4 mmol) and ethylenediamine (16.9 g, 281 mmol) were stirred at 110°C for 18 hours, the reaction product was washed with methanol and then filtered. The solvent was removed from the filtrate by distillation, and the resulting residue was purified by recrystallization using hexane. N-Docosanoylethylenediamine was obtained as a white crystal (yield of 65%, 14.0 g, 36.7 mmol).

In a N,N-dimethylformamide (40 mL) solution of N-docosanoylethylenediamine (12.0 g, 31.4 mmol) and triethylamine (6.35 g, 62.8 mmol), succinic anhydride (3.45 g, 34.5 mmol) was added over 10 minutes, and the mixture was stirred at 100°C for 15 minutes. After succinic anhydride was dissolved, acetic anhydride (4.81 g, 47.1 mmol) was added dropwise to the reaction crude solution over 10 minutes, and the mixture was stirred at 100°C for 1 hour. The reaction mixture was poured into water (200 mL), and the precipitate was filtered and washed with water. The precipitate was purified by recrystallization using 2-propanol. N-Docosanoylaminoethylsuccinimide was obtained as a white crystalline powder (yield of 92%, 13.4 g, 28.9 mmol).

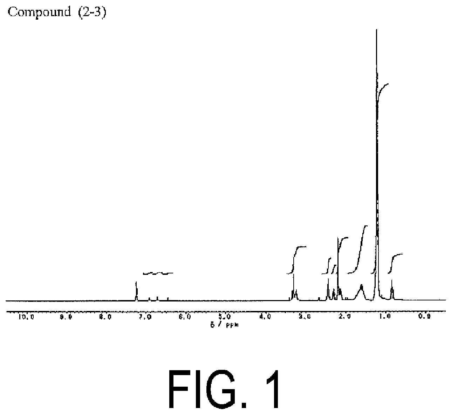

N-Docosanoylaminoethylsuccinimide (4.00 g, 8.60 mmol) and N,N-dimethyl-1,3-propanediamine (2.63 g, 25.8 mmol) were stirred at 120°C for 18 hours. The reaction mixture was poured into methanol, and the precipitate was filtered and washed with methanol. The resulting solid was purified by recrystallization using acetone and methanol. N-(Docosanoylaminoethylaminosuccinamoylaminopropyl)-N,N-dimethylamine (compound (2-3)) represented by Formula (2-3) below was obtained as a white crystalline powder (yield of 94%, 4.58 g, 8.08 mmol). A ¹H-NMR (CDCl₃) measurement result of the resulting compound is shown in FIG. 1.

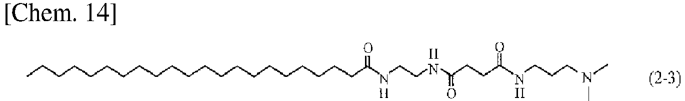

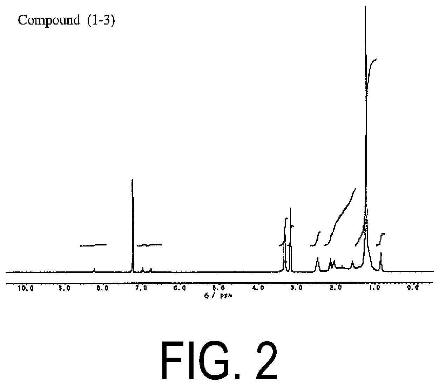

N-(Docosanoylaminoethylaminosuccinamoylaminopropyl)-N,N-dimethylamine (4.00 g, 7.06 mmol), a 35% hydrogen peroxide aqueous solution (2.06 mL), and 2-propanol (10 mL) were stirred at 60°C for 5 hours. Palladium carbon (about 10 mg) was added to the reaction solution, and the reaction solution was stirred at room temperature for 18 hours. The reaction solution was filtered, the solvent was distilled off, and then the residue was purified by column chromatography (silica gel, 2-propanol/methanol). N-(Docosanoylaminoethylaminosuccinamoylaminopropyl)-N,N-dimethylamine oxide (compound (1-3)) represented by Formula (1-3) below was obtained as a white solid (yield of 69%, 2.84 g, 4.87 mmol). A ¹H-NMR (CDCl₃) measurement result of the resulting compound is shown in FIG. 2.

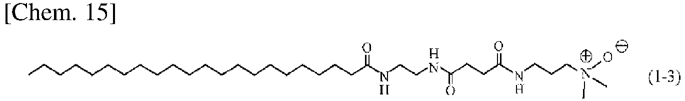

### Preparation Example 2: Production of compound (1-4)

N-Docosanoylaminoethylsuccinimide was obtained by the same method as in Preparation Example 1.

The resulting N-docosanoylaminoethylsuccinimide (8.00 g, 17.2 mmol) and hexamethylenediamine (10.0 g, 86.1 mmol) were stirred at 120°C for 18 hours. The reaction mixture was poured into methanol, and the precipitate was filtered and washed with methanol. The resulting solid was purified by recrystallization using acetonitrile and methanol. N-(Docosanoylaminoethyl)aminosuccinamoylaminohexylamine was obtained as a white crystalline powder (yield of 69%, 6.91 g, 11.9 mmol).

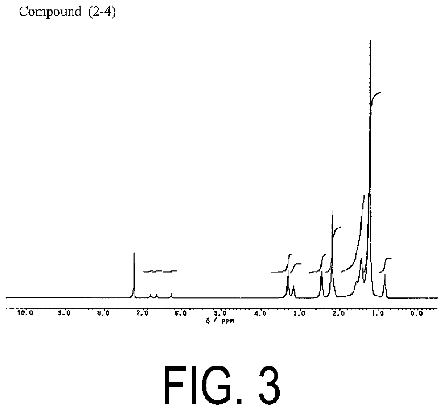

N-(Docosanoylaminoethyl)aminosuccinamoylaminohexylamine (3.25 g, 5.59 mmol), a 37% aqueous formaldehyde solution (2.73 mL), and formic acid (1.55 g, 33.7 mmol) were dissolved in 2-propanol (15 mL), and the solution was stirred at 100°C for 4 hours. The reaction mixture was poured into a 1 M aqueous sodium hydroxide solution (20 mL), and a crystal was filtered. The resulting crystal was recrystallized with methanol and acetone, and N-(docosanoylaminoethylaminosuccinamoylaminohexyl)-N,N-dimethylamine (compound (2-4)) represented by Formula (2-4) below was obtained as a white solid (yield of 89%, 3.03 g, 4.98 mmol). A ¹H-NMR (CDCl₃) measurement result of the resulting compound is shown in FIG. 3.

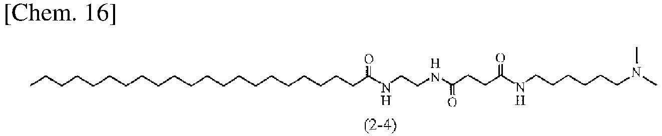

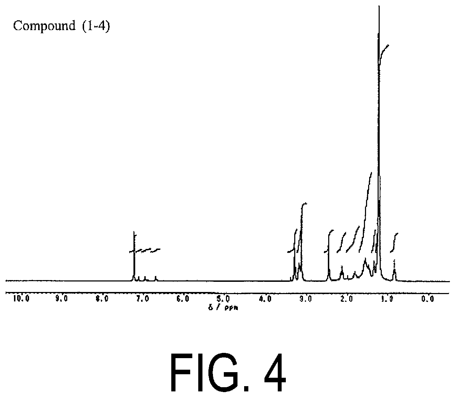

N-(Docosanoylaminoethylaminosuccinamoylaminohexyl)-N,N-dimethylamine (2.80 g, 4.60 mmol), a 35% hydrogen peroxide aqueous solution (1.30 mL), and 2-propanol (10 mL) were stirred at 60°C for 5 hours. Palladium carbon (about 10 mg) was added to the reaction solution, and the reaction solution was stirred at room temperature for 18 hours. The reaction solution was filtered, the solvent was distilled off, and then the residue was purified by column chromatography (silica gel, 2-propanol/methanol). N-(Docosanoylaminoethylaminosuccinamoylaminohexyl)-N,N-dimethylamine oxide (compound (1-4)) represented by Formula (1-4) below was obtained as a white solid (yield of 74%, 2.13 g, 3.40 mmol). A ¹H-NMR (CDCl₃) measurement result of the resulting compound is shown in FIG. 4.

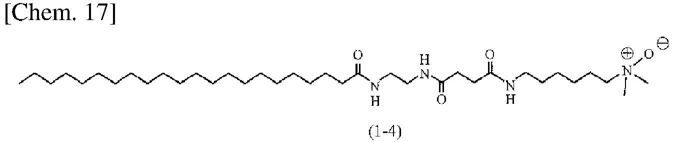

### Preparation Example 3: Production of compound (1-1)

Methyl eicosanoate (18.0 g, 55.1 mmol) and ethylenediamine (16.5 g, 276 mmol) were stirred at 110°C for 18 hours, and the reaction product was washed with methanol and then filtered. The solvent was removed from the filtrate by distillation, and the resulting residue was purified by recrystallization using hexane. N-Eicosanoylethylenediamine was obtained as a white crystal (yield of 68%, 13.3 g, 37.5 mmol).

In a N-dimethylformamide (30 mL) solution of N-eicosanoylethylenediamine (10.0 g, 28.2 mmol) and triethylamine (5.71 g, 56.4 mmol) succinic anhydride (3.10 g, 31.0 mmol) was added over 10 minutes, and the mixture was stirred at 100°C for 15 minutes. After succinic anhydride was dissolved, acetic anhydride (4.32 g, 42.3 mmol) was added dropwise to the reaction crude solution over 10 minutes, and the mixture was stirred at 100°C for 1 hour. The reaction mixture was poured into water (150 mL), and the precipitate was filtered and washed with water. The precipitate was purified by recrystallization using 2-propanol. N-Eicosanoylaminoethylsuccinimide was obtained as a 91% white crystalline powder at a yield (11.2 g, 25.7 mmol).

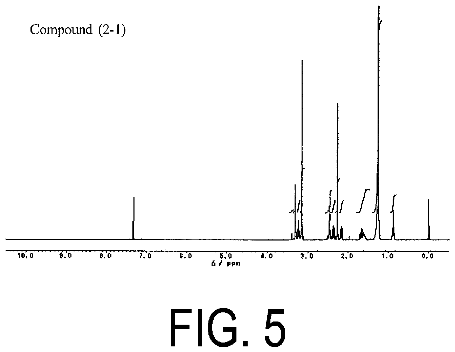

N-Eicosanoylaminoethylsuccinimide (4.00 g, 9.16 mmol) and N,N-dimethyl-1,3-propanediamine (2.81 g, 27.5 mmol) were stirred at 120°C for 18 hours. The reaction mixture was poured into methanol, and the precipitate was filtered and washed with methanol. The resulting solid was purified by recrystallization using acetone and methanol. N-(Eicosanoylaminoethylaminosuccinamoylaminopropyl)-N,N-dimethylamine (compound (2-1)) represented by Formula (2-1) below was obtained as a white crystalline powder (yield of 91%, 4.49 g, 8.34 mmol). A ¹H-NMR (CDCl₃) measurement result of the resulting compound is shown in FIG. 5.

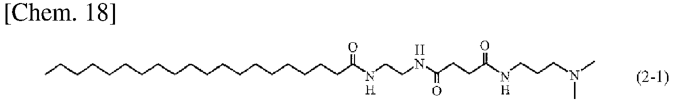

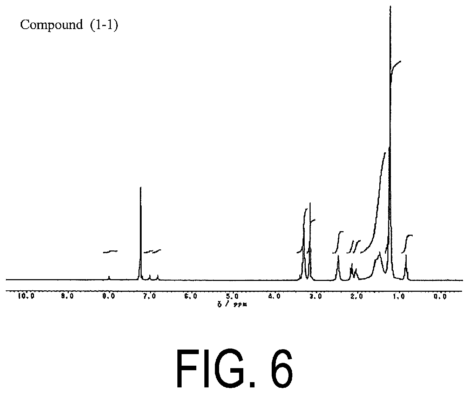

N-(Eicosanoylaminoethylaminosuccinamoylaminopropyl)-N,N-dimethylamine (4.00 g, 7.42 mmol), a 35% hydrogen peroxide aqueous solution (2.16 mL), and 2-propanol (10 mL) were stirred at 60°C for 5 hours. Palladium carbon (about 10 mg) was added to the reaction solution, and the reaction solution was stirred at room temperature for 18 hours. The reaction solution was filtered, the solvent was distilled off, and then the residue was purified by column chromatography (silica gel, 2-propanol/methanol). N-(Eicosanoylaminoethylaminosuccinamoylaminopropyl)-N,N-dimethylamine oxide (compound (1-1)) represented by Formula (1-1) below was obtained as a white solid (yield of 58%, 2.39 g, 4.30 mmol). A ¹H-NMR (CDCl₃) measurement result of the resulting compound is shown in FIG. 6.

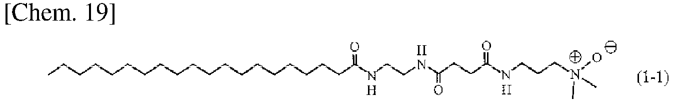

### Preparation Example 4: Production of compound (1-5)

A compound represented by Formula (2-5) below was obtained in the same manner as in Preparation Example 2 except that methyl octadecanoate was used instead of methyl docosanoate; and a compound represented by Formula (1-5) below was obtained.

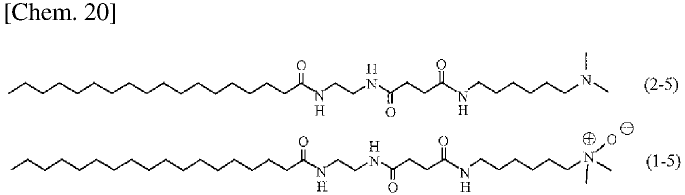

### Preparation Example 5: Production of compound (1-6)

A compound represented by Formula (2-6) below was obtained in the same manner as in Preparation Example 1 except that methyl octadecanoate was used instead of methyl docosanoate; and a compound represented by Formula (1-6) below was obtained.

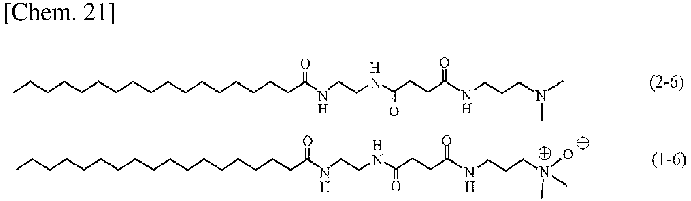

### Preparation Example 6: Production of compound (1-7)

A compound represented by Formula (2-7) below was obtained in the same manner as in Preparation Example 2 except that methyl palmitate was used instead of methyl docosanoate; and a compound represented by Formula (1-7) below was obtained.

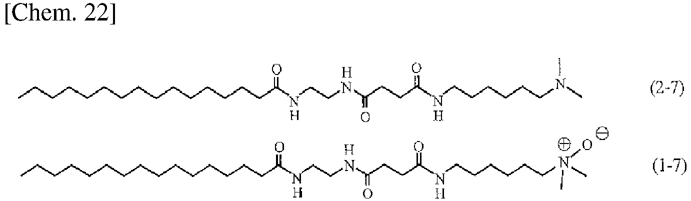

### Preparation Example 7: Production of compound (1-8)

A compound represented by Formula (2-8) below was obtained in the same manner as in Preparation Example 1 except that methyl palmitate was used instead of methyl docosanoate; and a compound represented by Formula (1-8) below was obtained.

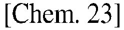

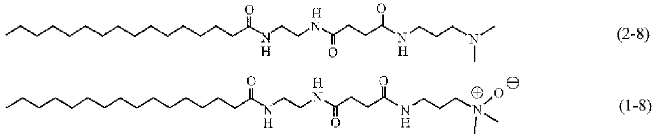

### Preparation Example 8: Production of compound (1-9)

A compound represented by Formula (2-9) below was obtained in the same manner as in Preparation Example 2 except that methyl myristate was used instead of methyl docosanoate and octamethylenediamine was used instead of hexamethylenediamine; and a compound represented by Formula (1-9) below was obtained.

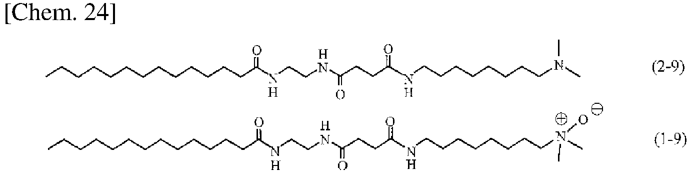

### Examples 1 to 10 and Comparative Examples 1 to 4: Production of composition

Each of compounds (1-1), and (1-3) to (1-9) obtained in Preparation Examples 1 to 8 above, which were thickening stabilizers, ethyl cellulose (EC), and a fluid organic material (1-methyl-amino-2,3-propanediol (MAPD), 1-amino-2,3-propanediol (1APD), or 1,2-propanediol (PD) were mixed according to the formulations shown in Table 1, dissolved by heating to form a miscible material, then cooled to 25°C, and a composition was obtained.

Combinations of each of compounds (1-1), (1-3) to (1-9) with 1-methyl-amino-2,3-propanediol (MAPD) or 1-amino-2,3-propanediol (1APD) were each stirred with heating at 100°C for 1 hour and a miscible material was formed.

A combination of ethyl cellulose (EC) with 1,2-propanediol (PD) was stirred with heating at 80°C for 24 hours and dissolved.

For ethyl cellulose (EC), "ETHOCEL STD 200" available from Nisshin Kasei Co., Ltd. was used.

The viscosity of the resulting composition was measured by changing a shear rate in a log scale from 0.1 to 100 s⁻¹ using a steady flow viscosity measurement mode at 25°C using a viscosity/viscoelasticity measuring device (rheometer) (trade name "RheoStress 600", available from HAAKE) equipped with a cone-plate sensor (a cone angle of 1° was used for a diameter of 60 mm, and cone angles of 1°, 2°, and 4° were used for a diameter of 35 mm) and a Peltier temperature controller.

### Evaluation of thickening properties

The viscosity was evaluated according to the criteria below from the viscosity of the resulting composition at a shear rate of 0.1 s⁻¹. The larger value means higher viscosity and thus indicates that the content of the compound (1) for making the composition moderately viscous can be further reduced.
1: 5 Pa·s or lower
2: Higher than 5 Pa·s and 10 Pa·s or lower
3: Higher than 10 Pa·s and 50 Pa·s or lower
4: Higher than 50 Pa·s

In addition, residual ash contents at 250°C of the compositions obtained in Examples and Comparative Examples were measured by the following method.

The temperature of 5 mg each of the compositions was increased from 20°C to 400°C at 10°C/min using a TG-DTA, and the residual ash amount at 250°C was measured. The proportion of the residual ash amount relative to the total amount of the composition was calculated as the residual ash content (mass%).

The results are summarized and shown in Table 1 below.

Examples 1 to 8 contained from 0.3 to 0.5 mass% of the thickening stabilizer compound, and thus all had both an excellent viscosity of 3 to 4 and a low residual ash content of 0.2 to 0.4 mass%.

Examples 9 and 10 contained 1 mass% of the thickening stabilizer compound, and thus both Examples had an excellent viscosity of 3 to 4 as well as a low residual ash content of 0.7 to 0.9 mass%.

Comparative Examples 1 and 2 contained 1 mass% of the thickening stabilizer compound but had a poor viscosity of 1 to 2.

Comparative Examples 3 and 4 contained 5 mass% of the thickening stabilizer compound but had a low viscosity of 2 to 3 and a very high residual ash content of 4.9 mass%.

### [Table 1]

**Table 1**

| (mass%) | Thickening stabilizer | | | | | | | | | Fluid organic materials | | | Viscosity | Residual ash content (mass%) |
|---|---|---|---|---|---|---|---|---|---|---|---|---|---|---|
| | Compound (1-4) | Compound (1-3) | Compound (1-1) | Compound (1-5) | Compound (1-6) | Compound (1-9) | Compound (1-7) | Compound (1-8) | EC | MAPD | 1APD | PD | | |
| Example 1 | 0.3 | 0 | 0 | 0 | 0 | 0 | 0 | 0 | 0 | 99.7 | 0 | 0 | 4 | 0.2 |
| Example 2 | 0.4 | 0 | 0 | 0 | 0 | 0 | 0 | 0 | 0 | 0 | 99.6 | 0 | 4 | 0.3 |
| Example 3 | 0 | 0.4 | 0 | 0 | 0 | 0 | 0 | 0 | 0 | 99.6 | 0 | 0 | 4 | 0.3 |
| Example 4 | 0 | 0.5 | 0 | 0 | 0 | 0 | 0 | 0 | 0 | 0 | 99.5 | 0 | 4 | 0.3 |
| Example 5 | 0 | 0 | 0.5 | 0 | 0 | 0 | 0 | 0 | 0 | 0 | 99.5 | 0 | 3 | 0.3 |
| Example 6 | 0 | 0 | 0 | 0.5 | 0 | 0 | 0 | 0 | 0 | 0 | 99.5 | 0 | 3 | 0.4 |
| Example 7 | 0 | 0 | 0 | 0 | 0.5 | 0 | 0 | 0 | 0 | 0 | 99.5 | 0 | 3 | 0.3 |
| Example 8 | 0 | 0 | 0 | 0 | 0 | 0.5 | 0 | 0 | 0 | 0 | 99.5 | 0 | 3 | 0.4 |
| Example 9 | 0 | 0 | 0 | 0 | 0 | 0 | 1 | 0 | 0 | 0 | 99 | 0 | 4 | 0.7 |
| Example 10 | 0 | 0 | 0 | 0 | 0 | 0 | 0 | 1 | 0 | 0 | 99 | 0 | 3 | 0.9 |
| Comparative Example 1 | 0 | 0 | 0 | 0 | 0 | 0 | 1 | 0 | 0 | 0 | 0 | 99 | 2 | 0.7 |
| Comparative Example 2 | 0 | 0 | 0 | 0 | 0 | 0 | 0 | 1 | 0 | 0 | 0 | 99 | 1 | 0.9 |
| Comparative Example 3 | 0 | 0 | 0 | 0 | 0 | 0 | 0 | 0 | 5 | 0 | 95 | 0 | 3 | 4.9 |
| Comparative Example 4 | 0 | 0 | 0 | 0 | 0 | 0 | 0 | 0 | 5 | 0 | 0 | 95 | 2 | 4.9 |

To summarize the above, configurations of the present disclosure and their variations will be described in addition below.
[1] A composition containing a miscible material of a compound (1) represented by Formula (1) and a compound (A) represented by Formula (A).
[2] The composition according to [1], in which R¹ in Formula (1) above is a linear alkyl group having from 11 to 13 or from 17 to 21 carbons.
[3] The composition according to [1] or [2], in which R² and R³ in Formula (1) above are identical or different and represent a linear alkylene group having from 2 or 4 carbons.
[4] The composition according to any one of [1] to [3], in which R⁴ in Formula (1) above is a linear alkylene group having from 1 to 8 carbons.
[5] The composition according to [3], in which R¹ in Formula (1) above is a linear alkyl group having from 11 to 21 carbons and R⁴ is a linear alkylene group having from 1 to 8 carbons.
[6] The composition according to [3], in which R¹ in Formula (1) above is a linear alkyl group having from 11 to 13 carbons and R⁴ is a linear alkylene group having from 4 to 8 carbons; R¹ is a linear alkyl group having 17 carbons and R⁴ is a linear alkylene group having from 1 to 5 carbons; or R¹ is a linear alkyl group having from 18 to 21 carbons and R⁴ is a linear alkylene group having from 4 to 8 carbons.
[7] The composition according to [3], in which R¹ in Formula (1) above is a linear alkyl group having from 18 to 21 carbons and R⁴ is a linear alkylene group having from 4 to 8 carbons.
[8] The composition according to any one of [1] to [7], in which R⁵ and R⁶ in Formula (1) above are methyl groups.
[9] The composition according to [1], in which R¹ in Formula (1) above is a linear alkyl group having from 11 to 13 carbons, R² and R³ are linear alkylene groups having 2 or 4 carbons, R⁴ is a linear alkylene group having from 1 to 8 carbons, and R⁵ and R⁶ are methyl groups.
[10] The composition according to [1], in which R¹ in Formula (1) above is a linear alkyl grousi p having from 17 to 21 carbons, R² and R³ are linear alkylene groups having 2 or 4 carbons, R⁴ is a linear alkylene group having from 1 to 8 carbons, and R⁵ and R⁶ are methyl groups.
[11] The composition according to [1], in which R¹ in Formula (1) above is a linear alkyl group having from 11 to 21 carbons, R² and R³ are linear alkylene groups having 2 or 4 carbons, R⁴ is a linear alkylene group having from 1 to 8 carbons, and R⁵ and R⁶ are methyl groups.
[12] The composition according to [1], in which R¹ in Formula (1) above is a linear alkyl group having from 11 to 13 carbons, R² and R³ are linear alkylene groups having 2 or 4 carbons, R⁴ is a linear alkylene group having from 4 to 8 carbons, and R⁵ and R⁶ are methyl groups.
[13] The composition according to [1], in which R¹ in Formula (1) above is a linear alkyl group having 17 carbons, R² and R³ are linear alkylene groups having 2 or 4 carbons, R⁴ is a linear alkylene group having from 1 to 5 carbons, and R⁵ and R⁶ are methyl groups.
[14] The composition according to [1], in which R¹ in Formula (1) above is a linear alkyl group having from 18 to 21 carbons, R² and R³ are linear alkylene groups having 2 or 4 carbons, R⁴ is a linear alkylene group having from 4 to 8 carbons, and R⁵ and R⁶ are methyl groups.
[15] The composition according to [1], in which the compound (1) is at least one compound selected from compounds represented by Formulas (1-1) to (1-9).
[16] The composition according to any one of [1] to [15], in which R^{b} in Formula (A) above is a trimethylene group.
[17] The composition according to any one of [1] to [16], in which R^{a}, R^{b}, or R^{c} in Formula (A) above may have a total of 0 to 2 amino groups and/or hydroxyl groups.
[18] The composition according to any one of [1] to [16], in which R^{c} in Formula (A) above is a hydrogen atom.
[19] The composition according to any one of [1] to [15], in which the compound (A) is a compound (A') represented by Formula (A').
[20] The composition according to [19], in which the compound (A') is at least one selected from 1-amino-2,3-propanediol and 1-methylamino-2,3-propanediol.
[21] The composition according to any one of [1] to [20], further containing an electrical property-imparting material.
[22] The composition according to [21], in which the electrical property-imparting material is a conductive metal material or a magnetic material.
[23] The composition according to [22], in which a content of the conductive metal material or the magnetic material is from 97 to 99.9 wt.%.
[24] The composition according to [21], in which a content of the electrical property-imparting material is from 0.1 to 3 wt.%.
[25] The composition according to any one of [1] to [24], in which viscosity [viscosity (η) at 25°C and a shear rate of 0.1 s⁻¹] measured with a rheometer is from 10 to 100 Pa·s.
[26] A method for producing a composition, in which the composition described in any one of [1] to [25] is obtained through mixing a compound (1) represented by Formula (1) and a compound (A) represented by Formula (A) to form a miscible material.

### Industrial Applicability

The composition according to the present disclosure has moderate viscosity for application properties and ejection properties and can be fired at low temperatures, and thus is suitably used for forming a wiring and an electrode of an electronic device by a printing method. Thus, the present disclosure has industrial applicability.

## Claims

1. A composition comprising a miscible material of:
a compound (1) and a compound (A), the compound (1) being represented by Formula (1): 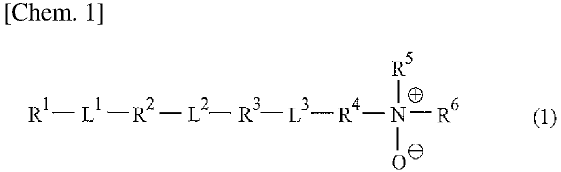
wherein R¹ represents a monovalent linear aliphatic hydrocarbon group having from 10 to 25 carbons; R² and R³ are identical or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbons; R⁵ and R⁶ are identical or different and represent a monovalent aliphatic hydrocarbon group having from 1 to 3 carbons or a hydroxyalkyl ether group; L¹ to L³ represent an amide bond; when L¹ and L³ are -CONH-, L² is -NHCO-; and when L¹ and L³ are -NHCO-, L² is -CONH-; and
the compound (A) being represented by Formula (A): 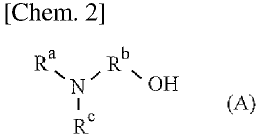
wherein R^{a} and R^{c} are identical or different and represent a hydrogen atom or a monovalent aliphatic hydrocarbon group having from 1 to 12 carbons and may have a substituent; R^{b} represents a divalent aliphatic hydrocarbon group having from 1 to 12 carbons and may have a substituent; and the substituents are each an amino group and/or a hydroxyl group.

2. The composition according to claim 1, wherein R^{c} in Formula (A) is a hydrogen atom.

3. The composition according to claim 2, wherein the compound (A) is a compound (A') represented by Formula (A'): 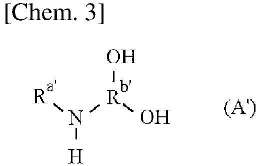 where R^{a'} represents a monovalent aliphatic hydrocarbon group having from 1 to 12 carbons; and R^{b'} represents a trivalent aliphatic hydrocarbon group having from 1 to 12 carbons.

4. The composition according to any one of claims 1 to 3, further comprising an electrical property-imparting material.

5. A method for producing a composition, wherein the composition described in any one of claims 1 to 4 is formed through mixing a compound (1) represented by Formula (1) and a compound (A) represented by Formula (A) to form a miscible material: 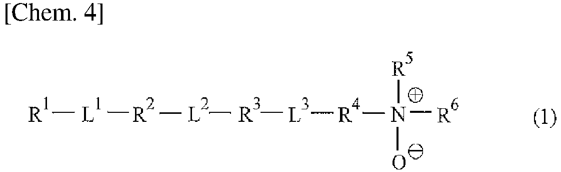 where R¹ represents a monovalent linear aliphatic hydrocarbon group having from 10 to 25 carbons; R² and R³ are identical or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbons; R⁵ and R⁶ are identical or different and represent a monovalent aliphatic hydrocarbon group having from 1 to 3 carbons or a hydroxyalkyl ether group; L¹ to L³ represent an amide bond; when L¹ and L³ are -CONH-, L² is -NHCO-; and when L¹ and L³ are -NHCO-, L² is -CONH-; and 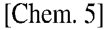 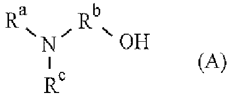 where R^{a} and R^{c} are identical or different and represent a hydrogen atom or a monovalent aliphatic hydrocarbon group that has from 1 to 12 carbons and may have a substituent; R^{b} represents a divalent aliphatic hydrocarbon group that has from 1 to 12 carbons and may have a substituent; and the substituents are each an amino group and/or a hydroxyl group.
